# EUROPEAN PATENT APPLICATION

(11) **EP 2 930 638 A1**
(43) Date of publication of application: **14.10.2015**
(21) Application number: 15162881.5
(22) Date of filing: 09.04.2015
(51) Int. Cl.: G06F 19/00

(54) **A METHOD AND A PROCEDURE TO REPLICATE VISUAL ACUITY DEFICIENCIES FOR USE IN ACCESSIBILITY VERIFICATION**

(30) Priority: 10.04.2014 US 201461978111 P; 01.04.2015 US 201514676629
(71) Applicant: Synchronoss Technologies, Inc., Bridgewater, NJ 08807 (US)
(72) Inventor: Paul, Sumeet S., Evanston, IL Illinois 60202 (US)
(74) Representative: Goddar, Heinz J.

(57) **Abstract**

Embodiments of the present invention relate to a replicator that reproduces acuity deficiencies for use during an accessibility verification procedure. The replicator applies in real time at least one filter to an original video feed. Each filter includes a programmable definition to mimic a type of visual acuity limitation, such as color blindness, myopia, hyperopia, astigmatism or reduced contrast. Prior to and/or during the accessibility verification procedure, a tester is able to select one or more filters to be applied to the original video feed to thereby produce a filtered video feed. The filtered video feed is shown on a display and portrays what an individual with corresponding visual disorder(s) sees. The replicator can be updated to add, delete or modify one or more definitions.

## Description

### Related Applications

This application claims benefit of priority under 35 U.S.C. section 119(e) of the copending United States Provisional Patent Application Serial No. 61/978,111, filed April 10, 2014, entitled "METHOD AND PROCEDURE TO REPLICATE VISUAL ACUITY DEFICIENCIES FOR USE IN ACCESSIBILITY VERIFICATION," which is hereby incorporated by reference in its entirety.

### Field of Invention

The present invention relates to an application under test (AUT). More particularly, the present invention relates to a method and a procedure to replicate visual acuity deficiencies for use in accessibility verification of an AUT.

### Background of the Invention

During software development and production, various ADA (Americans with Disabilities Act) guidelines must be met. Those guidelines require specific capabilities of any product to be accessible by individuals with various limitations. However, during the design and testing of new products, such as applications, it is rare that an organization has someone who has those actual limitations. This results in the necessity to call on trained professionals in the accessibility space to define or design user interfaces to meet the requirements. Yet, when it comes to testing the implementations, the quality assurance groups are generally forced to simply verify implementation against the documented design, and not actually to verify compliance against the documented minimums in terms of color limitations, text size, etc. There are a variety of organizations that can be called upon to perform external validation of products, but all testing and results tend to be subjective, including when run by individuals with those limitations.

### Brief Summary of the Invention

Embodiments of the present invention relate to a replicator that reproduces acuity deficiencies for use during an accessibility verification procedure. The replicator applies in real time at least one filter to an original video feed. Each filter includes a programmable definition to mimic a type of visual acuity limitation, such as color blindness, myopia, hyperopia, astigmatism or reduced contrast. Prior to and/or during the accessibility verification procedure, a tester is able to select one or more filters to be applied to the original video feed to thereby produce a filtered video feed. The filtered video feed is shown on a display and portrays what an individual with corresponding visual disorder(s) sees. The replicator can be updated to add, delete or modify one or more definitions.

In one aspect, a device is provided. The device includes a camera for receiving an original video feed, and an acuity deficiencies replicator configured to receive the original video feed and to apply at least one filter to the original video feed to obtain a filtered video feed. The device also includes a screen for displaying the filtered video feed.

In some embodiments, the original video feed is of at least an application under test (AUT). In some embodiments, the camera and the screen are located on opposite sides of the device, and the AUT is in a field of view of the camera.

In some embodiments, the device further includes a non-transitory memory for storing an application, and a processing component coupled to the memory, the processing component configured for processing the application. In some embodiments, the application is the acuity deficiencies replicator.

In some embodiments, the at least one filter is associated with a type of visual acuity limitation. For example, the type of visual acuity limitation is color blindness, myopia, hyperopia, astigmatism and reduced contrast.

In some embodiments, the acuity deficiencies replicator is also configured to generate a file of two views of an image, wherein a first of the two views is an unfiltered view of the image and a second of the two views is a filtered view of the image according to the at least one filter. In some embodiments, the acuity deficiencies replicator is also configured to communicate with a defect tracking system such that the generated file is submitted to the defect tracking system.

In another aspect, a non-transitory computer-readable medium is provided. The non-transitory computer-readable medium stores instructions that, when executed by a computing device, cause the computing device to perform a method. The method includes receiving a first set of selections, wherein each selection in the first set of selections pertains to a type of visual acuity limitation, obtaining a first video feed, applying, according to the first set of selections, a corresponding first set of filters to the first video feed, and outputting a second video feed that is different from the first video feed.

In some embodiments, the first video feed is of an application under test (AUT).

In some embodiments, the first set of filters applied to the first video feed results in the second video feed.

In some embodiments, the first video feed is a live video feed that is captured by a camera that is communicatively coupled with the computing device, wherein the first set of filters is applied in realtime to the first video feed such that the second video feed is output in realtime to a screen that is communicatively coupled with the computing device.

In some embodiments, the method further includes receiving a second set of selections, and applying, according to the second set of selections, a corresponding second set of filters to the first video feed. In some embodiments, the second set of filters applied to the first video feed results in the second video feed.

In some embodiments, the method further includes, prior to the step of receiving a first set of selections, receiving a plurality definitions, wherein each of the definitions is an algorithm for replicating a visual acuity limitation that is presented to a user as a selection. In some embodiments, when the selection for the visual acuity limitation is activated, the algorithm is applied to the first video feed.

In yet another aspect, a system is provided. The system includes an application under test (AUT), and a first computing device. The first computing device includes a camera for receiving an original video feed of the AUT, and an acuity deficiencies replicator configured to receive the original video feed of the AUT and to apply at least one filter to the original video feed of the AUT to obtain a filtered video feed of the AUT. The first computing device also includes a screen for displaying the filtered video feed of the AUT.

In some embodiments, the system further includes a second computing device including the AUT executing thereon, and a mount configured to couple with the first computing device such that the first computing device is at a predetermined distance away from the second computing device.

In some embodiments, the system further includes a defect tracking system that is communicatively coupled with the first computing device, wherein at least a portion of the filtered video feed of the AUT is transmitted from the first computing device to the defect tracking system.

### Brief Description of the Drawings

The foregoing will be apparent from the following more particular description of example embodiments of the invention, as illustrated in the accompanying drawings in which like reference characters refer to the same parts throughout the different views. The drawings are not necessarily to scale, emphasis instead being placed upon illustrating embodiments of the present invention.
Figure 1 illustrates a block diagram of an exemplary computing device in accordance with some embodiments.
Figure 2 illustrates an exemplary system in accordance with some embodiments.
Figure 3 illustrates an exemplary method in accordance with some embodiments.

### Detailed Description of the Invention

In the following description, numerous details are set forth for purposes of explanation. However, one of ordinary skill in the art will realize that the invention can be practiced without the use of these specific details. Thus, the present invention is not intended to be limited to the embodiments shown but is to be accorded the widest scope consistent with the principles and features described herein.

Embodiments of the present invention relate to a replicator that reproduces acuity deficiencies for use during an accessibility verification procedure. The replicator applies in real time at least one filter to an original video feed. Each filter includes a programmable definition to mimic a type of visual acuity limitation, such as color blindness, myopia, hyperopia, astigmatism or reduced contrast. Prior to and/or during the accessibility verification procedure, a tester is able to select one or more filters to be applied to the original video feed to thereby produce a filtered video feed. The filtered video feed is shown on a display and portrays what an individual with corresponding visual disorder(s) sees. The replicator can be updated to add, delete or modify one or more definitions.

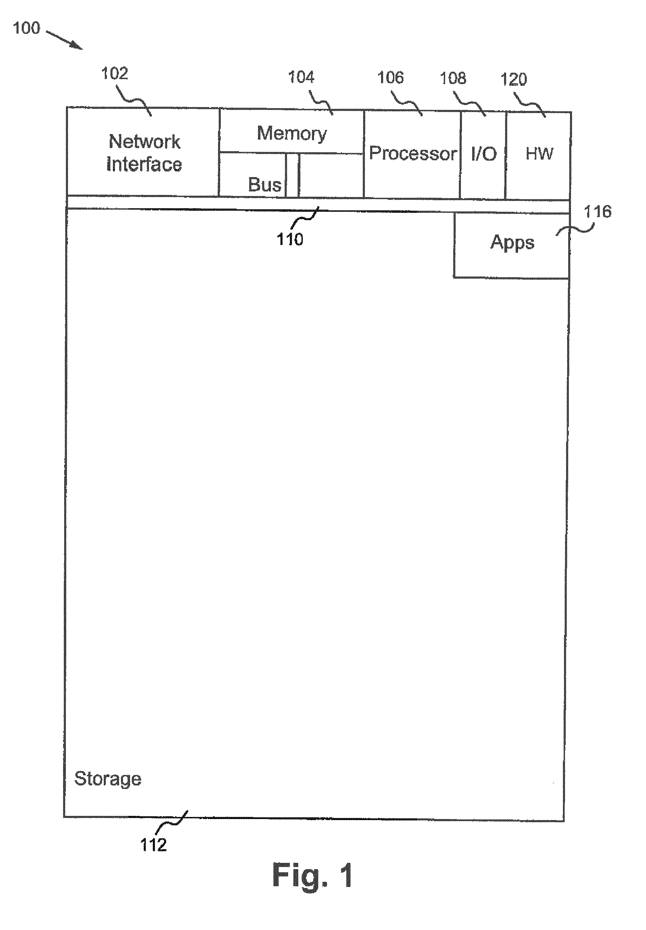

Figure 1 illustrates a block diagram of an exemplary computing device 100 in accordance with some embodiments. The computing device 100 is able to be used to acquire, cache, store, compute, search, transfer, communicate and/or display information. In general, a hardware structure suitable for implementing the computing device 100 includes a network interface 102, a memory 104, processor(s) 106, I/O device(s) 108, a bus 110 and a storage device 112. The choice of processor 106 is not critical as long as a suitable processor with sufficient speed is chosen. In some embodiments, the computing device 100 includes a plurality of processors 106. The memory 104 is able to be any conventional computer memory known in the art. The storage device 112 is able to include a hard drive, CDROM, CDRW, DVD, DVDRW, flash memory card, RAM, ROM, EPROM, EEPROM or any other storage device. The computing device 100 is able to include one or more network interfaces 102. An example of a network interface includes a network card connected to an Ethernet or other type of LAN. The I/O device(s) 108 are able to include one or more of the following: keyboard, mouse, monitor, display, printer, modem, touchscreen, button interface and other devices. Application(s) 114, such as an acuity deficiencies replicator, are likely to be stored in the storage device 112 and memory 104 and are processed by the processor 106. More or less components shown in Figure 1 are able to be included with the computing device 100. For example, the computing device 100 can include a camera. In some embodiments, the camera and the display screen are located on opposite sides of the computing device 100. Alternatively, the camera and the display screen are located on the same side of the computing device 100. The camera can be a built-in camera (native to the computing device) or a separate, disjoint camera. The computing device 100 can be a tablet, a mobile phone, a smart phone, a desktop computer, a laptop computer, a netbook, or any suitable computing device such as special purpose devices.

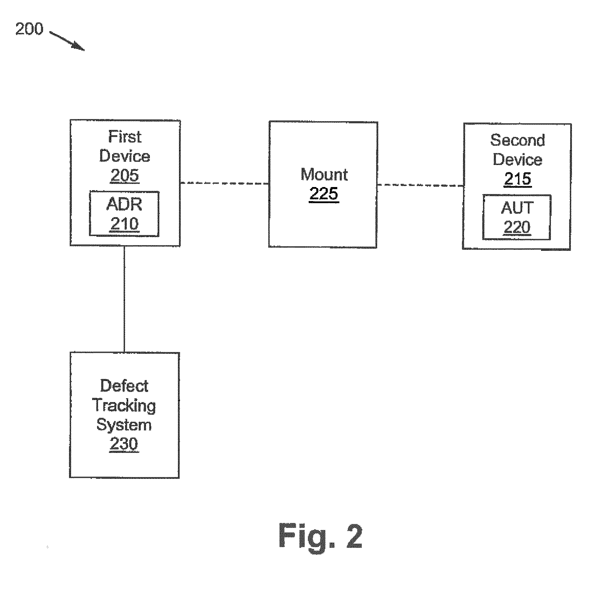

Figure 2 illustrates an exemplary system 200 in accordance with some embodiments. The system 200 includes a first computing device 205 and a second computing device 215. Each of the first computing device 205 and the second computing device 215 can be similarly configured as the computing device 100 of Figure 1. The system 200 also includes an acuity deficiencies replicator 210 and an application under test (AUT) 220. The replicator 210 is an application that reproduces acuity deficiencies for use during an accessibility verification procedure. The replication 210 typically executes or runs on the first computing device 205. The first computing device 215 is communicatively coupled with a screen and a camera.

The AUT 220 is an application that is examined, during an accessibility verification procedure, for barriers to accessibility and fixed accordingly prior to public release of the AUT 220. An exemplary AUT 220 is a web site that includes one or more web pages. The web pages should be developed such that it can be read by all users, including those with disabilities. The AUT 220 typically executes or runs on the second computing device 225. The second computing device 225 can be a device under test (DUT).

During an accessibility verification procedure, the first computing device 205 and the second computing device 215 are typically positioned at predetermined distance away from each other. The first computing device 205 can be physically held by a tester a predetermined distance away from the second computing device 215 during the accessibility verification procedure. Alternatively, a mount 225 can be used to couple with the first computing device such that the first computing device 205 is at the predetermined distance away from the second computing device 215 during the accessibility verification procedure. In some embodiments, the mount 225 includes a coupler and a stand, with the coupler configured to couple with the first computing device and the stand for positioning the mount 225 on a surface. In some embodiments, the mount 225 includes two couplers, with a first coupler configured to couple with the first computing device and the second coupler for coupling with the second computing device. During the accessibility verification procedure, the entire second computing device 215 is in a field of view of the camera of the first computing device 205 and is shown on the screen of the first computing device 205. Alternatively, only an interface of the second computing device 215 (showing the AUT 210) is in a field of view of the camera of the first computing device 205 and is shown on the screen of the first computing device 205.

In some embodiments, the first computing device 205 includes the camera for receiving an original video feed of the AUT 220, the acuity deficiencies replicator 210 configured to receive the original video feed of the AUT 220 and to apply at least one filter to the original video feed of the AUT 220 to obtain a filtered video feed of the AUT 220, and the screen for displaying the filtered video feed of the AUT 220. The tester views the AUT 220 on the screen of the first computing device 205. The acuity deficiency replicator 210 includes automated algorithms to replicate visual acuity limitations and to measure font size, button size and spacing, OCR readability, etc. The filtered video feed displayed on the screen of the first computing device 205 allows the tester to see what an individual with particular limitations sees. In other words, the acuity deficiency replicator 210 allows the tester to see through the eyes of the visually impaired.

The first computing device 205 can be communicatively coupled with a defect tracking system 230 such that at least a portion of the filtered video feed of the AUT 220 can be transmitted to the defect tracking system 230 automatically or on demand. A corresponding portion of the original video feed can also be transmitted along with the portion of the filtered video feed of the AUT 220 to the defect tracking system 230 for further comparison and documentation prior to the release of the AUT 220. In some embodiments, the defect tracking system 230 includes tools configured to record, report, manage and track issues. Although the first computing device 205 and the defect tracking system 230 are shown as separate, disjoint elements in Figure 2, the first computing device 205 and the defect tracking system 230 can within be a single element. In some embodiments, the defect tracking system 230 is implemented with acuity deficiencies replicator 210.

Prior to an accessibility verification procedure, a user (such as a developer or a tester) programs the acuity deficiencies replicator 210 with a plurality of definitions, wherein each of the definitions is an algorithm for replicating a visual acuity limitation that is presented as a selection during the accessibility verification procedure. In some embodiments, the algorithm includes one or more instructions for manipulating each pixel of each frame of the original video feed. The acuity deficiencies replicator 210 can be updated to add, delete or modify one or more definitions. In some embodiments, the acuity deficiencies replication 210 includes preprogrammed rules and/or use-defined rules for determining characteristics or properties of different elements (e.g., text, buttons, etc.) in the original video feed.

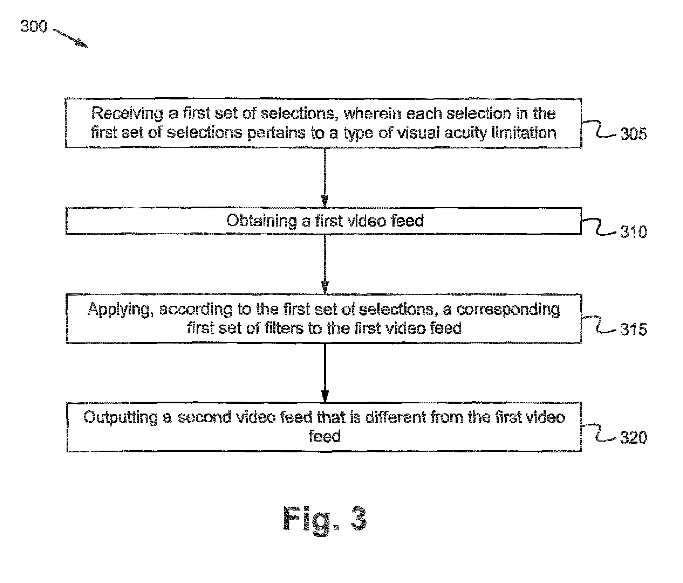

Figure 3 illustrates an exemplary method 300 in accordance with some embodiments. The method 300 is typically performed by an acuity deficiencies replicator, such as the acuity deficiencies replicator 210 of Figure 2, executing on a first computing device, such as the first computing device 205 of Figure 2, during an accessibility verification procedure. The first computing device is typically positioned at a predetermined distance away from a second computing device, such as the second computing device 215 of Figure 2. Assume that the acuity deficiencies replicator includes a plurality of definitions for mimicking visual limitations.

At a Step 305, a first set of selections is received by the acuity deficiencies replicator. Typically, the first set of selections is made by a tester. Each selection in the first set of selections pertains to a type of visual acuity limitation. Exemplary types of visual acuity limitation include different degrees of color blindness, myopia, hyperopia, astigmatism, etc. The first set of selections includes one or more selections.

At a Step 310, a first video feed is obtained by the acuity deficiencies replicator. In some embodiments, the first video feed is of an application under test (AUT) that is executing on the second computing device. In some embodiments, the first video feed is a live video feed of the AUT that is obtained by a camera that is communicatively coupled with the first computing device. Alternatively, the first video feed is a previously stored video feed stored on the first computing device and retrieved during the accessibility verification procedure.

At a Step 315, according to the first set of selections, a corresponding first set of filters is applied to the first video feed by the acuity deficiencies replicator. Typically, when a selection for a visual acuity limitation is activated, a corresponding algorithm is automatically applied to the first video feed. For example, upon a selection of red-green color blindness, values of pixels in each frame of the first video feed that are red or green are changed to a single shade of red-green by the acuity deficiencies replicator.

At a Step 320, a second video feed that is different from the first video feed is output by the acuity deficiencies replicator. In some embodiments, the first set of filters applied to the first video feed results in the second video feed. In some embodiments, the second video feed is output in realtime (e.g., as it comes) to a screen that is communicatively coupled with the first computing device.

The tester is able to test a different visual limitation or a different combination of visual limitations during the accessibility verification procedure. As such, a second set of selections can be received by the acuity deficiencies replicator during the accessibility verification procedure. The second set of selections is made by the tester. According to the second set of selections, a corresponding set second of filters is applied to the first video feed by the acuity deficiencies replicator. The second set of filters applied to the first video feed results in the second video feed, which is output in realtime to the screen.

In some embodiments, the acuity deficiencies replicator is also configured to generate a image file of two side-by-side views of an image. An image can be a still image from the first video feed or a previously stored image file. A first of the two views is an unfiltered view of the image and a second of the two views is a filtered view of the image. In some embodiments, the acuity deficiencies replicator is also configured to communicate with a defect tracking system, such as the defect tracking system 230 of Figure 2, so that the generated image file is submitted to the defect tracking system for further comparison and documentation prior to the release of the AUT.

For example, if a tester were testing for red-green color blindness, the tester, using a first computing device, could select to apply a corresponding filter to an original live video stream of images of an AUT that are displayed on a second computing device (e.g., DUT). These images would be shown converting all reds and greens to a single shade of red-green on the first computing device. If this results in a situation in which the AUT does not meet the ADA requirements, the tester could submit an image file into a defect tracking system, wherein the image file includes an unfiltered view of an image and a filtered view of the image. It should be understood that the same process could be used to, for example, "fuzz" an image to replicate an individual not wearing corrective lenses or an individual who has other issues, such as night blindness.

In some embodiments, not only can the acuity deficiencies replicator apply filters to a video stream to reproduces acuity deficiencies, the acuity deficiencies replicator is able to perform a number of tests, including one that determines whether text in an AUT has an allowable font size (e.g, at least minimum font size and not larger than maximum font size) and one that determines whether linkable elements, such as buttons, are appropriately spaced apart. During an accessibility verification procedure, a tester inputs the distance between a first computing device and a second computing device, and/or other necessary configurations such that the acuity deficiencies replicator can perform computations to determine any online barriers that may exists. Barriers or issues, such as improper font size and improper positioning of linkable elements, are pointed out by the acuity deficiencies replicator in realtime. As discussed above, an image file showing any of these issues can be submitted to the defect tracking system for further comparison and documentation prior to the release of the AUT.

As demonstrated above, by using the acuity deficiencies replicator on a first computing device to view an interface of a second computing device, a tester (such as a quality assurance individual) can have an objective bar to judge the solution under test against. In addition, the tester would then be able to capture a portion of the second video feed that does not conform along with the corresponding original video feed being viewed in a single image file that could then be transmitted as part of a bug report to a defect tracking system or, alternatively or in addition to, to other destination(s), thereby eliminating reporting discrepancies. The acuity deficiencies replicator allows a quality assurance (QA) organization to hire less experienced and less expensive QA staff as it removes the need for independent critical thinking from the process of deciding if there is an issue or not.

One of ordinary skill in the art will realize other uses and advantages also exist. While the invention has been described with reference to numerous specific details, one of ordinary skill in the art will recognize that the invention can be embodied in other specific forms without departing from the spirit of the invention. Thus, one of ordinary skill in the art will understand that the invention is not to be limited by the foregoing illustrative details, but rather is to be defined by the appended claims.

## Claims

1. A device comprising:
a camera for receiving an original video feed;
an acuity deficiencies replicator configured to:
receive the original video feed; and
apply at least one filter to the original video feed to obtain a filtered video feed; and
a screen for displaying the filtered video feed.

2. The device of Claim 1, wherein the original video feed is of at least an application under test (AUT).

3. The device of Claim 2, wherein the camera and the screen are located on opposite sides of the device, and wherein the AUT is in a field of view of the camera.

4. The device of Claim 1, further comprising:
a non-transitory memory for storing an application; and
a processing component coupled to the memory, the processing component configured for processing the application, wherein the application is the acuity deficiencies replicator.

5. The device of Claim 1, wherein the at least one filter is associated with a type of visual acuity limitation.

6. The device of Claim 5, wherein the type of visual acuity limitation is one of color blindness, myopia, hyperopia, astigmatism and reduced contrast.

7. The device of Claim 1, wherein the acuity deficiencies replicator is also configured to generate a file of two views of an image, wherein a first of the two views is an unfiltered view of the image and a second of the two views is a filtered view of the image according to the at least one filter.

8. The device of Claim 7, wherein the acuity deficiencies replicator is also configured to communicate with a defect tracking system such that the generated file is submitted to the defect tracking system.

9. A non-transitory computer-readable medium storing instructions that, when executed by a computing device, cause the computing device to perform a method, the method comprising:
receiving a first set of selections, wherein each selection in the first set of selections pertains to a type of visual acuity limitation;
obtaining a first video feed;
applying, according to the first set of selections, a corresponding first set of filters to the first video feed; and
outputting a second video feed that is different from the first video feed.

10. The non-transitory computer-readable medium of Claim 9, wherein the first set of filters applied to the first video feed results in the second video feed.

11. The non-transitory computer-readable medium of Claim 9, wherein the first video feed is a live video feed that is captured by a camera that is communicatively coupled with the computing device, wherein the first set of filters is applied in realtime to the first video feed such that the second video feed is output in realtime to a screen that is communicatively coupled with the computing device.

12. The non-transitory computer-readable medium of Claim 9, wherein the method further comprises:
receiving a second set of selections; and
applying, according to the second set of selections, a corresponding second set of filters to the first video feed, wherein the second set of filters applied to the first video feed results in the second video feed.

13. The non-transitory computer-readable medium of Claim 9, wherein the method further comprises, prior to the step of receiving a first set of selections, receiving a plurality definitions, wherein each of the definitions is an algorithm for replicating a visual acuity limitation that is presented to a user as a selection, wherein when the selection for the visual acuity limitation is activated, the algorithm is applied to the first video feed.

14. A system comprising:
a. an application under test (AUT); and
b. a first computing device including:
a camera for receiving an original video feed of the AUT;
an acuity deficiencies replicator configured to:
receive the original video feed of the AUT; and
apply at least one filter to the original video feed of the AUT to obtain a filtered video feed of the AUT; and
a screen for displaying the filtered video feed of the AUT.

15. The system of Claim 14, further comprising:
a second computing device including the AUT executing thereon;
a mount configured to couple with the first computing device such that the first computing device is at a predetermined distance away from the second computing device; and
a defect tracking system that is communicatively coupled with the first computing device, wherein at least a portion of the filtered video feed of the AUT is transmitted from the first computing device to the defect tracking system.
